(19) Europäisches Patentamt
European Patent Office
Office européen des brevets

(11) EP 1 317 441 B1

(12) EUROPÄISCHE PATENTSCHRIFT

(45) Veröffentlichungstag und Bekanntmachung des Hinweises auf die Patenterteilung:
**15.06.2005 Patentblatt 2005/24**

(21) Anmeldenummer: **01984639.3**

(22) Anmeldetag: **12.09.2001**

(51) Int Cl.[7]: **C07D 335/02**, A01N 43/14

(86) Internationale Anmeldenummer:
**PCT/EP2001/010538**

(87) Internationale Veröffentlichungsnummer:
**WO 2002/022596 (21.03.2002 Gazette 2002/12)**

# (54) VERFAHREN ZUR HERSTELLUNG EINES CYCLOHEXENONOXIMETHER-LITHIUMSALZES

METHOD FOR PRODUCING A CYCLOHEXENONE OXIME ETHER LITHIUM SALT

PROCEDE POUR PREPARER UN SEL DE LITHIUM CYCLOHEXENONOXIMETHER

(84) Benannte Vertragsstaaten:
**CH DE FR GB LI**

(30) Priorität: **13.09.2000 DE 10045131**

(43) Veröffentlichungstag der Anmeldung:
**11.06.2003 Patentblatt 2003/24**

(73) Patentinhaber: **BASF AKTIENGESELLSCHAFT
67056 Ludwigshafen (DE)**

(72) Erfinder:
- **MAYWALD, Volker
67071 Ludwigshafen (DE)**
- **KOBER, Reiner
67136 Fussgönheim (DE)**
- **HEIMANN, Frank
67065 Ludwigshafen (DE)**
- **OBERDORF, Klaus
69117 Heidelberg (DE)**
- **HARREUS, Albrecht
67063 Ludwigshafen (DE)**
- **GÖTZ, Norbert
67547 Worms (DE)**
- **VOSSEN, Marcus
68199 Mannheim (DE)**

(74) Vertreter: **Wolter, Thomas, Dr. et al
Reitstötter, Kinzebach & Partner (GbR)
Patentanwälte
Postfach 86 06 49
81633 München (DE)**

(56) Entgegenhaltungen:
**DE-A- 19 545 212**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 317 441 B1

Printed by Jouve, 75001 PARIS (FR)

## Beschreibung

Die vorliegende Erfindung betrifft ein Verfahren zur Herstellung von 2-{1-[2-(4-Chlorphenoxy)-propoxyimino]-butyl}-3-hydroxy-5-(tetrahydrothiopyran-3-yl)-cyclohex-2-enon-Lithiumsalz aus der entsprechenden Säure. Die mit diesem Verfahren erhältlichen Produkte, deren Verwendung sowie Pflanzenschutzmittel, die derartige Produkte enthalten, werden ebenfalls beschrieben.

Cyclohexenonoximether und deren Metallsalze sind als wertvolle Pflanzenschutzmittel allgemein bekannt. Sie werden vor allem als Gräser-Herbizide geschätzt. Als besonders geeignet haben sich Metallsalze (WO 97/20807) und insbesondere das Cyclohexenonoximether-Lithiumsalz der Formel (I) erwiesen:

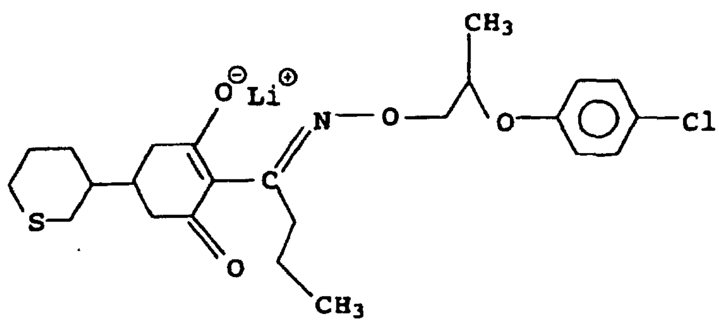

(I)

Neben der Optimierung der stofflichen Eigenschaften kommt mit Blick auf eine industrielle Produktion und Anwendung dieses Wirkstoffs auch der Entwicklung eines effizienten Herstellungsverfahrens besondere Bedeutung zu.

Für das vorstehend beschriebene Lithiumsalz verweist die WO 97/20807 zunächst auf die EP 456 112, wonach die entsprechende freie Säure 2-{1-[2-(4-Chlorphenoxy)-propoxyimino]-butyl}-3-hydroxy-5-(tetrahydrothiopyran-3-yl)-cyclohex-2-enon in gelöster Form anfällt und dann ohne Isolierung zum Lithiumsalz umgesetzt werden kann.

In Anlehnung an die allgemein üblichen Angaben zur Herstellung solcher Metallsalze durch Umsetzung der freien Säure mit einem Hydrid, Hydroxid, Alkoholat oder Carbonat des gewünschten Metallions schildert die WO 97/20807 beispielhaft die Herstellung des Li-Salzes durch Umsetzung einer Lösung des Cyclohexenonoximethers in Toluol mit einer wässrigen Lithiumhydroxidlösung und anschließender Abtrennung des entstandenen Feststoffanteils. Ferner wird in einem weiteren Beispiel das Lithium-Salz durch Natrium/Lithium-Austausch hergestellt. Hierbei wird so vorgegangen, dass man eine wässrige Lösung des entsprechenden Cyclohexenonoximether-Natrium-Salzes mit einer wässrigen Lithiumchloridlösung zur Reaktion bringt und anschließend den Feststoffanteil abtrennt.

Das zuerst genannte Verfahren stößt bei der Abtrennung des entstandenen Feststoffanteils auf Probleme. Ungünstige Filtrationseigenschaften, insbesondere hohe und schwankende Filterwiderstände machen es außerordentlich schwierig, den Feststoff im Technikums- und Produktionsmaßstab mit üblichen Filtrationsorganen wie Drucknutschen oder Zentrifugen zu isolieren.

Der Nachteil des auf Natrium/Lithium-Austausch basierenden Verfahrens ist offensichtlich: Der freie Cyclohexenonoximether muß zunächst in einem mit Ausbeuteverlusten verbundenen zusätzlichen Schritt in das Natriumsalz überführt werden.

Die sich daraus ergebende Aufgabe, ein vor allem im Hinblick auf den Filtrationsvorgang, Ausbeute und Reinheit effizientes Verfahren zur Herstellung des Cyclohexenonoximether-Lithium-Salzes zu finden, wird erfindungsgemäß dadurch gelöst, dass man ein bestimmtes Lösungsmittelgemisch als Reaktionsmedium verwendet.

Gegenstand der vorliegenden Erfindung ist daher ein Verfahren zur Herstellung von 2-{1-[2-(4-Chlorphenoxy)-propoxyimino]-butyl}-3-hydroxy-5-(tetrahydrothiopyran-3-yl)-cyclohex-2-enon-Lithiumsalz der Formel (I),

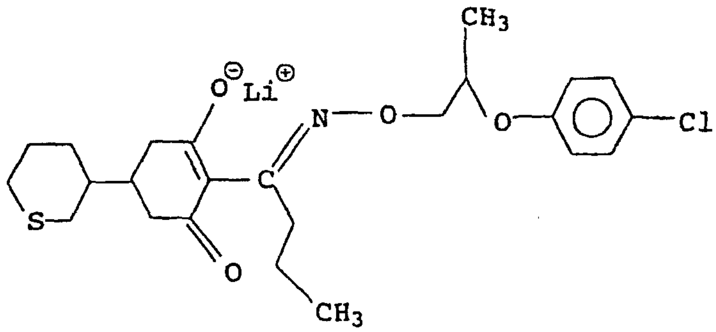

(I)

durch Umsetzung von 2-{1-[2-(4-Chlorphenoxy)-propoxyimino]-butyl}-3-hydroxy-5-(tetrahydrothiopyran-3-yl)-cyclohex-2-enon der Formel (II)

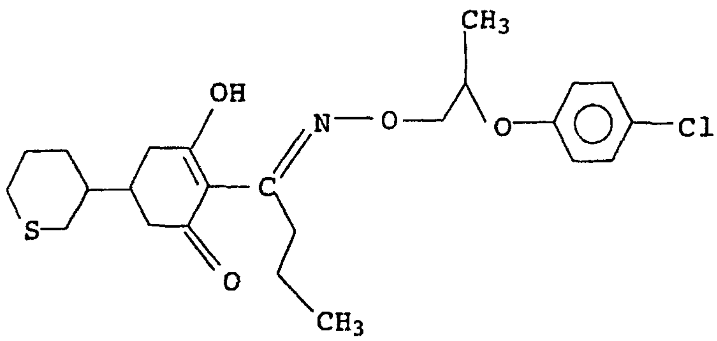

(II)

und Lithiumhydroxid in einem Lösungsmittelgemisch sowie Gewinnung der Verbindung der Formel (I), das dadurch gekennzeichnet ist, dass das Lösungsmittelgemisch Methanol und wenigstens einen aromatischen Kohlenwasserstoff enthält und vor Gewinnung wenigstens ein Teil des Lösungsmittels entfernt wird.

Das erfindungsgemäße Verfahren gestattet überraschenderweise eine vergleichsweise effizientere Gewinnung des Verfahrensprodukts.

Die hier gewählte Darstellung der Verbindungen der Formeln (I) und (II) schließt isomere Formen dieser Verbindungen mit ein. Insbesondere zu nennen sind geometrische und Stereoisomere, wie cis/trans-Isomere, Enantiomere oder Diastereoisomere, sowie Tautomere, die im vorliegenden Fall vor allem auf die Enolstruktur zurückzuführen sind. Demnach können insbesondere die Verbindungen der Formel (II) auch als Cyclohexan-1,3-dion-Derivate beschrieben werden. Neben den im wesentlichen reinen Isomeren gehören zu den Verbindungen der Formeln (I) und (II) auch deren Isomerengemische, z.B. Stereoisomerengemische.

Das Ausgangsprodukt des erfindungsgemäßen Verfahrens, der Cyclohexenonoximether 2-{1-[2-(4-Chlorphenoxy)-propoxyimino]-butyl}-3-hydroxy-5-(tetrahydrothiopyran-3-yl)-cyclohex-2-enon obiger Formel (II), kann beispielsweise nach den in der WO 97/20807 und EP 456 112 beschriebenen Verfahren erhalten werden. Demnach kann diese Verbindung in an sich bekannter Weise aus 2-(1-Butyloxy)-3-hydroxy-5-(tetrahydrothiopyran-3-yl)-cyclohex-2-enon der Formel (III)

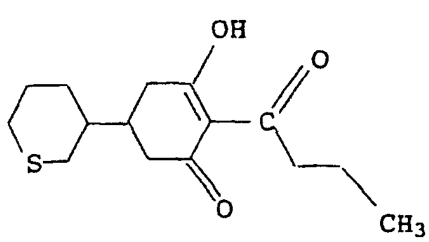

(III)

und dem Hydroxylamin 1-Aminooxy-2-(4-chlorphenoxy)-propan der Formel (IV)

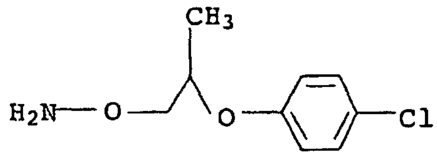

(IV)

erhalten werden. Eine mögliche Art der Umsetzung ist z.B. in der EP 169 521 beschrieben. Die Verbindungen der Formeln (III) und (IV) können auch in Form ihrer Salze eingesetzt werden, insbesondere Basenadditionssalze von (III) und Säureadditionssalze von (IV). Als salzbildende Ionen kommen die üblichen in Frage, beispielsweise Metallionen und insbesondere Alkali- und Erdalkalimetallionen, vor allem Natrium, für (III) sowie Halogenide und vor allem Sulfate und Hydrogensulfate für (IV). Die Umsetzung erfolgt vorzugsweise in wässrigem Reaktionsmedium.

Angaben zur Synthese der Verbindung der Formel (III) sind insbesondere den in der EP 456 112 genannten Dokumenten zu entnehmen. Auf die relevanten Ausführungen wird hier in vollem Umfang Bezug genommen. Die Synthese der Verbindung der Formel (IV) kann in Anlehnung an Houben-Weyl, 10/1 S. 1181 ff. erfolgen.

Im Rahmen ihrer Synthese kann die Verbindung der Formel (II) nach üblicher Aufarbeitung des Reaktionsgemisches isoliert werden. Als Ausgangsprodukt für das erfindungsgemäße Verfahren können aber auch auf der Verbindung der Formel (II) basierende Gemische eingesetzt werden, die bei der Synthese anfallen.

So setzt man gemäß einer im Hinblick auf das erfindungsgemäße Verfahren besonderen Ausführungsform die Verbindung der Formel (III) oder ein Salz davon mit der Verbindung der Formel (IV) oder ein Salz davon um und verteilt das Reaktionsprodukt in ein organisches Lösungsmittel oder Lösungsmittelgemisch, das vorzugsweise einen und insbesondere den erfindungsgemäß zu verwendenden aromatischen Kohlenwasserstoff, enthält. Dazu kann das organische Lösungsmittel oder Lösungsmittelgemisch bereits während oder im Anschluß an die Reaktion dem vorzugsweise wässrigen Reaktionsmedium zugesetzt werden. Wasser oder ein Wasser/Toluol-Gemisch ist als Reaktionsmedium bevorzugt. Erforderlichenfalls wird der pH-Wert so eingestellt, daß das Reaktionsprodukt der Formel (II) in dem organischen Lösungsmittel oder Lösungsmittelgemisch löslich ist. Als zweckmäßig hat sich ein pH-Wert von etwa 5 bis 6 erwiesen.

So kann als Ausgangsmaterial des erfindungsgemäßen Verfahrens eine Lösung der Verbindung der Formel (II), vorzugsweise in aromatischem Kohlenwasserstoff und insbesondere in Toluol eingesetzt werden. Bei dieser Lösung kann es sich beispielsweise um die erforderlichenfalls eingeengte oder mit weiterem Lösungsmittel versetzte organische Phase des Reaktionsgemisches aus obiger Umsetzung der Verbindungen (III) und (IV) handeln. Es kann sich aber auch um die nach Beendigung der zu Verbindung (II) führenden Umsetzung zur Extraktion des Reaktionsgemisches oder des nach dem Einengen des Reaktionsgemisches resultierenden Rückstandes verwendete organischen Phase handeln, die durch Einengen oder Zusatz von weiterem Lösungsmittel auf eine erwünschte Konzentration von (II) eingestellt werden kann.

Ein besonderer Gegenstand der vorliegenden Erfindung ist daher ein Verfahren zur Herstellung von (I) aus (III) und (IV) über eine Lösung von (II), ohne (II) in Substanz zu isolieren.

Erfindungsgemäß findet die Umsetzung von (II) mit Lithiumhydroxid in einem Lösungsmittelgemisch statt, das Methanol und wenigstens einen aromatischen Kohlenwasserstoff enthält.

Zweck des aromatischen Kohlenwasserstoffes oder Kohlenwasserstoffgemisches ist es, die Verbindung (II) zu lösen. Zu brauchbaren aromatischen Kohlenwasserstoffen gehören insbesondere Lösungsmittel der Benzolreihe, wie alkylsubstituierte Benzole, z.B. Toluol und Xylol. Vor allem kommt Toluol in Betracht.

Fällt die Verbindung der Formel (II) in isolierter Form an, so kann zunächst wenigstens ein Teil der Substanz in dem aromatischen Kohlenwasserstoff(gemisch) gelöst und dann als Lösung mit den übrigen Reaktanten in Kontakt gebracht werden. Gegebenenfalls kann auch Substanz, also beispielsweise die Gesamtmenge an (II) oder eine Teilmenge davon zu einem bereits aromatischen Kohlenwasserstoff enthaltenden Reaktionsgemisch zugegeben werden. Fällt die Verbindung der Formel (II) bereits als Lösung an, so kann diese Lösung verwendet werden, sofern das Lösungsmittel die erfindungsgemäße Umsetzung nicht stört. Dabei kann es erforderlich sein, erfindungsgemäß zu verwendenden aromatischen Kohlenwasserstoff dieser Lösung oder dem Reaktionsgemisch zuzusetzen. Auch ein Entfernen wenigstens eines Teils des mit der Lösung von Verbindung (II) anfallenden Lösungsmittels, gegebenenfalls unter gleichzeitigem Zusatz von aromatischem Kohlenwasserstoff, kann sinnvoll sein.

Vorzugsweise setzt man eine Lösung von 2-{1-[2-(4-Chlorphenoxy)-propyloximino]-butyl}-5-tetrahydrothiopyran-3-yl-3-hydroxycyclohex-2-en-1-on der Formel (II) um, die wenigstens einem aromatischen Kohlenwasserstoff enthält. Demnach ist wenigstens ein Teil der Verbindung der Formel (II) in einem aromatischen Kohlenwasserstoff oder einem aromatischen Kohlenwasserstoffgemisch gelöst. Verwendet wird eine Lösung der Verbindung (II), die auf wenigstens einem aromatischen Kohlenwasserstoff basiert, neben diesem aber auch andere Lösungsmittel enthalten kann. Gemäß einer besonderen Ausführungsform handelt es sich um eine Lösung der insgesamt umzusetzenden Menge an Verbindung (II) in einem aromatischen Kohlenwasserstoff, vorzugsweise Toluol.

Es kann es von Vorteil sein, die Konzentration des Cyclohexenonoximethers (II) auf einen gewünschten Wert einzustellen. Setzt man eine Lösung der Verbindung (II) ein, so beträgt die Konzentration der Verbindung der Formel (II) vorzugsweise 5 bis 40 Gew.-%, vorzugsweise 10 bis 30 Gew.-% und insbesondere etwa 20 Gew.-%, bezogen auf das Gesamtgewicht der einzusetzenden Lösung. So betrifft eine bevorzugte Ausführungsform der vorliegenden Erfindung die Verwendung einer entsprechenden Toluol-Lösung, die insbesondere aus einem Vorkonzentrat mit höherer Konzentration an Cyclohexenonoximether (II) durch Zugabe von Lösungsmittel erhältlich ist.

Lithiumhydroxid (LiOH) fällt in der Regel als Feststoff an. Es kann beispielsweise in wasserfreier Form oder als Hydrat, insbesondere Monohydrat, eingesetzt werden. Handelsüblich sind z.B. kalziniertes Lithiumhydroxid oder Lithiumhydroxid-Monohydrat. Diese Substanzen werden üblicherweise mit einer chemischen Reinheit von wenigstens 98% angeboten. Aus wirtschaftlichen Gründen kommt insbesondere der Einsatz des Lithiumhydroxid-Monohydrats in Betracht. Wenigstens ein Teil des Feststoffs kann zunächst in Methanol, einem methanolhaltigen Lösungsmittel oder einem anderen Lösungsmittel, z.B. Wasser, gelöst und dann als Lösung mit den übrigen Reaktanten in Kontakt gebracht werden. Gegebenenfalls kann auch Feststoff, also beispielsweise die Gesamtmenge an LiOH oder eine Teilmenge davon als Feststoff zu einem bereits Methanol enthaltenden Reaktionsgemisch zugegeben werden.

Vorzugsweise setzt man eine Lösung von LiOH um, die Methanol enthält. Wenigstens ein Teil des LiOH ist demnach in Methanol oder einem methanolhaltigen Lösungsmittelgemisch gelöst. Verwendet wird eine Lösung von LiOH, die auf Methanol basiert, neben diesem aber auch andere Lösungsmittel enthalten kann. Bevorzugt sind methanolische LiOH-Lösungen, die neben möglichen weiteren Lösungsmitteln wenigstens 70 Gew.-%, vorzugsweise wenigstens 80 Gew.-% und insbesondere wenigstens 85 Gew.-% Methanol, bezogen auf die Gesamtmenge an Lösungmittel der zu verwendenden LiOH-Lösung, enthalten. Weitere mögliche Lösungsmittel sind insbesondere Wasser und niedere Alkohole, wie Ethanol, Propanol und Isopropanol. Gemäß einer besonderen Ausführungsform handelt es sich um eine Lösung der insgesamt umzusetzenden Menge an LiOH in Methanol.

Eine methanolische LiOH-Lösung enthält zweckmäßigerweise 1 bis 7 Gew.-%, vorzugsweise 2 bis 6 Gew.-% und insbesondere etwa 5 Gew.-% LiOH. Die Mengenangaben beziehen sich auf das Gesamtgewicht der eingesetzten Lösung vor Zugabe.

Das Cyclohexenonoximether-Li-Salz (I) wird durch Umsetzung von Cyclohexenonoximether (II) mit Lithiumhydroxid erhalten. Dazu sind zumindest Teile der Reaktanten gelöst.

Wie vorstehend ausgeführt kann es reaktionstechnisch von Vorteil sein, zunächst entsprechende Lösungen herzustellen und die Lösungen dann miteinander in Kontakt zu bringen, also in der Regel miteinander zu vermischen. Es ist aber auch möglich, geeignete Lösungen quasi in situ zu erzeugen.

Zweckmäßigerweise geht man so vor, dass entweder der Cyclohexenonoximether (II), insbesondere als Lösung, vorgelegt und Lithiumhydroxid, insbesondere als Lösung, zugegeben wird oder umgekehrt Lithiumhydroxid vorgelegt und der Cyclohexenonoximether (II) zugegeben wird. Es können auch beide Reaktanten, insbesondere als Lösungen, gleichzeitig zusammengegeben werden, gegebenenfalls unter Vorlage eines Teils der jeweiligen Lösung (en) oder von Lösungsmittel. Die Zugabe kann auf einmal, portionsweise oder im kontinuierlichen Zulauf erfolgen. Es ergibt sich das erfindungsgemäße Lösungsmittelgemisch, das wenigstens einen aromatischen Kohlenwasserstoff, Methanol und gegebenenfalls weitere Lösungsmittel, z.B. Wasser, enthält. Im Reaktionsverlauf bildet sich (weiteres) Wasser.

Um eine vollständige Umsetzung sicherzustellen, wird die Lithiumhydroxid-Lösung üblicherweise im molaren Überschuss eingesetzt. Vorzugsweise wird ein molarer Überschuß von 0,5 bis 10% bezogen auf den als Ausgangsprodukt eingesetzten Cyclohexenonoximether (II) verwendet. Es kann aber auch sinnvoll sein, Lithiumhydroxid im Molverhältnis 1:1 mit dem Cyclohexenonoximether (II) umzusetzen. In diesem Fall kann man beispielweise die Zugabe von LiOH, insbesondere als Lithiumhydroxid-Lösung, mit einer geeigneten pH-Elektrode überwachen und solange zudosieren, bis der Äquivalenzpunkt erreicht ist.

Die Dosierzeiten für die jeweiligen Reaktionspartner liegen üblicherweise im Bereich von 10 Minuten bis 360 Minuten, vorzugsweise 10 Minuten bis 120 Minuten, insbesondere bei etwa 30 Minuten.

Nach beendeter Zugabe aller Reaktanten kann bis zur vollständigen Umsetzung weitergerührt werden.

Die Umsetzung wird üblicherweise bei Temperaturen von -20°C bis 60°C, bevorzugt 0-40°C, besonders bevorzugt 20-30°C durchgeführt.

Vor Gewinnung des Cyclohexenonoximethersalzes (I) wird wenigstens ein Teil des Lösungsmittelgemisches entfernt. Insbesondere wird Methanol aus dem Lösungsmittelgemisch entfernt. Dies erfolgt in der Regel zusammen mit anderen Komponeten des Lösungsmittelgemisches, insbesondere als Methanol/Toluol/Wasser-Gemisch.

Die Entfernung erfolgt vorzugsweise durch Destillation, üblicherweise bei Temperaturen zwischen 20 und 70°C, bevorzugt bei 30-60°C und besonders bevorzugt bei 40-50°C.

Die Destillation kann unter Normaldruck oder bevorzugt unter vermindertem Druck durchgeführt werden.

Gemäß einer bevorzugten Ausführungsform wird die Temperatur im Reaktor während der Destillation im wesentlichen konstant gehalten. Die Destillation eines Azeotrops, beispielsweise des erfindungsgemäß bevorzugten Methanol/Toluol/Wasser-Gemisches, erfordert in diesem Fall eine Variation des Drucks. Die Druckvariation erfolgt in an sich bekannter Weise durch den Fachmann.

Die Entfernung von Lösungsmittel dient vor allem dazu, die Menge an gelöstem Cyclohexenonoximether-Lithiumsalz (I) zu verringern. Cyclohexenonoximether-Lithiumsalz (I) fällt aus. Es bildet sich eine Suspension. Demnach kann es sinnvoll sein, soviel Lösungsmittel zu entfernen, daß die Verbindung der Formel (I) im wesentlichen vollständig ausfällt.

Einem besonderen Aspekt zufolge wird Methanol im wesentlichen vollständig aus dem Reaktionsgemisch entfernt. Dann kann die Entfernung von Lösungsmittel beendet werden. Es kann aber auch sinnvoll sein, die Entfernung bereits früher abzubrechen. Als zweckmäßig hat es sich erwiesen, nach beendeter Entfernung des Methanols neues Methanol der Suspension zuzusetzen. Die zuzusetzende Menge sollte so bemessen sein, daß möglichst wenig und vorzugsweise im wesentlichen kein Produkt wieder in Lösung geht. Typischerweise kann man 1 bis 100 g, vorzugsweise 5 bis 50 g und insbesondere 20 bis 35 g Methanol pro Mol Cyclohexenonoximether (II) zusetzen.

Die Gewinnung des Cyclohexenonoximether-Lithiumsalzes (I) erfolgt üblicherweise, indem man die resultierende Suspension einer Fest/Flüssig-Trennung unterzieht. Hierzu stehen dem Fachmann eine Vielzahl geeigneter Trennverfahren zur Verfügung, vor allem Sedimentations- und Filtrationsvorgänge. Bevorzugt werden Filtration, insbesondere Druckfiltration, Bandfiltration, vakuumfiltration, zentrifugation, und ähnliches. Vergleichsweise vorteilhaft erlaubt das erfindungsgemäße Verfahren die Durchführung von Filtrationvorgängen, da hier relativ niedrige Filterwiderstände auftreten und dadurch angemessene Filtrationszeiten errreicht werden können.

Eine Reinigung des abgetrennten Feststoffs, insbesondere in Form eines Filterkuchens, ist in der Regel zweckmäßig. Dazu kann mit einem geeigneten Lösungsmittel gewaschen werden. Als Lösungsmittel kommen aromatische Kohlenwasserstoffe wie Benzol, Toluol und Xylol, aliphatische Kohlenwasserstoffe wie Pentan, Hexan, Heptan, Petrolether, Ligroin und Cyclohexan, Halogenkohlenwasserstoffe wie Dichlormethan, Tetrachlorkohlenstoff und Chlorbenzol, Ether wie Diethylether Ethyl-n-propylether, Di-n-butylether, Diisopropylether, tert.-Butyl-methylether, Di-isoamylether, Cyclohexylmethylether, Ethylenglykoldimethylether, Diethylenglykoldimethylether, Tetrahydrofuran, Dioxan, Anisol und Phenetol, Ketone wie Aceton und Methylethylketon, Nitrile wie Acetonitril, Butyronitril und Benzonitril, Sulfoxide und Sulfone wie Dimethylsulfoxid und Sulfolan, Amide wie Formamid, Methylformamid und Dimethylformamid oder Alkohole wie Methanol, Ethanol, Propanol, iso-Propanol n-Butanol, iso-Butanol, sec.-Butanol oder tert.-Butanol in Betracht. Auch Gemische dieser Lösungsmittel können eingesetzt werden. Insbesondere kommt der bereits für die Umsetzung verwendete aromatische Kohlenwasserstoff in Betracht.

Das zum Waschen zu verwendende Lösungsmittel kann zunächst auch zum Ausspülen der Reaktionsvorrichtung, insbesondere des Behältnisses, genutzt werden und dann erforderlichenfalls portionsweise durch den Filterkuchen geführt werden.

In der Regel ist auch zweckmäßig, den abgetrennten Feststoff, insbesondere den Filterkuchen, zu trocknen. Dies kann üblicherweise in Vakuumtrockenschränken, Schaufeltrocknern und weiteren demselben Zweck dienenden Vorrichtungen erfolgen. Es ist aber auch möglich, den Filterkuchen direkt auf dem Filter zu trocknen. Dies kann beispielweise durch Trockenblasen mit erforderlichenfalls erwärmtem Inertgas wie Stickstoff oder Argon durchgeführt werden.

Zur vorliegenden Erfindung gehört auch das nach dem erfindungsgemäßen Verfahren erhältliche Produkt, also im wesentlichen 2-{1-[2-(4-Chlorphenoxy)-propoxyimino]-butyl}-3-hydroxy-5-(tetrahydrothiopyran-3-yl)-cyclohex-2-enon-Lithiumsalz der Formel (I). Der Ausdruck "im wesentlichen" meint erfindungsgemäß in der Regel ein prozentuales Verhältnis von wenigstens 90 %, vorzugsweise von wenigstens 95 % und insbesondere von wenigstens 98 %. In diesem Zusammenhang beschreibt der Ausdruck den Gehalt an 2-{1-[2-(4-Chlorphenoxy)-propoxyimino]-butyl}-3-hydroxy-5-(tetrahydrothiopyran-3-yl)-cyclohex-2-enon-Lithiumsalz der Formel (I) bezogen auf das Gesamtgewicht des erhältlichen Produktes. Eine besondere Form von erfindungsgemäß erhältlichem Produkt weist einen Gehalt an (I) von wenigstens 96 Gew.-%, vorzugsweise von wenigstens 97 Gew.-% und insbesondere von wenigstens 98 Gew.-%, bezogen auf das Gesamtgewicht des gewonnenen Produktes, auf. Vorteilhafterweise beträgt der Gehalt an organischen Verbindungen, die sich von der Verbindung der Formel (I) und im weiteren Sinne auch von Verbindungen der Formeln (II), (III) und/oder (IV) ableiten, weniger als 2 Gew.-% und vorzugsweise weniger als 1 Gew.-%.

Zur vorliegenden Erfindung gehören auch Pflanzenschutzmittel, die 2-{1-[2-(4-Chlorphenoxy)-propoxyimino]-butyl}-3-hydroxy-5-(tetrahydrothiopyran-3-yl)-cyclohex-2-enon-Lithiumsalz, herstellbar nach dem erfindungsgemäßen Verfahren, enthalten. Weiterhin können die Mittel je nach Anwendungszweck einen oder mehrere weitere Wirkstoffe, insbesondere Pflanzenschutzwirkstoffe, und/oder übliche Hilfsstoffe enthalten.

Das Cyclohexenonoximether-Lithiumsalz (I) bzw. die es enthaltenden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich dabei vor allem nach den Verwendungszwecken; sie sollten eine möglichst feine Verteilung des erfindungsgemäßen Salzes (I) gewährleisten.

Als inerte Hilfsstoffe kommen im wesentlichen in Betracht:

Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanole sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Lithiumsalz und gegebenenfalls weitere Wirkstoffe können in gelöster oder ungelöster, insbesondere fester, z.B. partikulärer Form in den Mitteln enthalten sein.

Die Konzentration des Wirkstoffs (I) in den anwendungsfertigen Zubereitungen kann in weiten Bereichen variiert werden, etwa von 0,001 bis 98 Gew.%, vorzugsweise 0,01 bis 95 Gew.%. Die Wirkstoffe werden dabei in einer Reinheit von 90% bis 100%, vorzugsweise 95% bis 100% (nach NMR-Spektrum) eingesetzt.

Gemäß einer besonderen Ausführungsform enthält das Pflanzenschutzmittel
1 bis 70 Gew.-% 2-{1-[2-(4-Chlorphenoxy)-propoxyimino]-butyl}-3-hydroxy-5-(tetrahydrothiopyran-3-yl)-cyclohex-2-enon-Lithiumsalz;
0 bis 40 Gew.-% wenigstens eines weiteren Wirkstoffs; und
0 bis 99 Gew.-% üblicher Hilfsstoffe.

Zur Verbreiterung des Wirkungsspektrums und zur Erzielung synergistischer Effekte kann das Cyclohexenonoximether-Lithiumsalz (I) mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-$CF_3$-Phenylderivate, Carbamate, Chinolincarbonsäure und deren Derivate, Chloracetanilide, weitere Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Besonders geeignet sind N-(Butoxymethyl)-2-chlor-N-(2,6-diethylphenyl)-acetamid (Common name: Butachlor), 2-(1,3-Benzthiazol-2-yloxy)-N-methyl-acetanilid (Common name: Mefenacet), 3,7-Dichlorchinolin-8-carbonsäure (Common name: Quinclorac), α-(4,6-Dimethoxypyrimidin-2-yl-carbamoyl-sulfamoyl)-o-toluolsäure-methylester (Common name: Bensulfuronmethyl), 3-Isopropyl-1*H*-2,1,3-benzothiadiazin-4-(3*H*)one-2,2-dioxide (Common name: Bentazon), N-(Ethylthio-carbonyl)-azepan (Common name: Molinate), N,N-Diethyl-carbaminsäure-4-chlorbenzylthioester (Common name: Thiobencarb), N-(2-Propoxyethyl)-2-chlor-N-(2,6-diethylphenyl)acetamid (Common name: Pretilachlor), 3,5-Bis(methylthio-carbonyl)-2-difluormethyl-4-(2-methylpropyl)-6-trifluormethyl-pyridin (Common name: Dithiopyr), 2-[4-(6-Chlor-benzoxazol-2-yloxy)-phenoxy]-propionsäure-ethylester (Common name: Fenoxapropethyl), N-(2-Phenyl-prop-2-yl-thiocarbonyl)-piperidin (Common name: Dimepiperate), 4-(2,4-Dichlorbenzoyl)-1,3-dimethylpyrazol-5-yl-toluyl-4-sulfonat (Common name: Pyrazolynate, Pyrazolate), 2-[4-(2,4-Dichlorbenzoyl)-1,3-dimethyl-pyrazol-5-yloxy]-acetophenon (Common name: Pyrazoxyfen), 2-[4-(2,4-Dichlor-m-toluyl)-1,3-dimethylpyrazol-5-yloxy]-4'-methylacetophenon (Common name: Benzofenap), 2-(2-Naphthyloxy)-propionanilid (Common name: Naproanilid), 5-(4,6-Dimethoxypyrimidin-2-yl-carbamoylsulfamoyl)-1-methylpyrazol-4-carbonsäuremethylester (Common name: Pyrazosulfuronethyl), 1-(4,6-Dimethoxy-1,3,5-triazin-2-yl)-3-[2-(2-methoxyethoxy)-phenylsulfonyl]-harnstoff (Common name: Cinosulfuron), 2-Brom-3,3-dimethyl-N-(1-methyl-1-phenylethyl)-butyramid (Common name: Bromobutide), 1-(1-Methyl-1-phenylethyl)-3-p-toluyl-harnstoff (Common name: Dymron, Daimuron), $N^2$-(1,2-Dimethylpropyl)-$N^4$-ethyl-6-methylthio-1,3,5-triazin-2,4-diamin (Common name: Dimethametryn), S-Benzyl-1,2-dimethylpropyl(ethyl)-thiocarbamat (Common name: Esprocarb), (Z)-N-But-2-enyloxymethyl-2-chlor-2',6'-diethylacetanilid (Common name: Butenachlor), S-2-Methylpiperidinocarbonylmethyl-O,O-dipropyl-phosphorodithionat (Common name: Piperophos), (1RS,2SR,4SR)-1,4-Epoxy-p-menth-2-yl-2-methylbenzylether (Common name: Cinmethylin), N-(3,4-Dichlorphenyl)-propanamid (Common name: Propanil), α-Chlor-N-(3-methoxy-2-thienyl)-methyl-2',6'-dimethylacetanilid, 4-Ethoxybenz-2',3'-dihydrochloranilid, 1-Diethylcarbamoyl-3-(2,4,6-trimethylphenylsulfonyl)-1,2,4-triazol, 3-(2-Chlorphenylmethyl)-1(1-methyl-1-phenylethyl)-harnstoff, 2-(2-Chlor-4-mesylbenzoyl)-cyclohexan-1,3-dion, 2,4-Dichlorphenoxyessigsäure (Common name: 2,4-D), N-(2-Chlorimidazol[1,2-a]pyridin-3-yl-sulfonyl)-N'-(4,6-dimethoxy-2-pyrimidyl)harnstoff (Common name: Imazosulfuron), 1-{[2-(Cyclopropylcarbonyl)phenyl]aminosulfonyl}-3-(4,6-dimethoxypyrimidin-2-yl) harnstoff, 1-(4,6-Dimethoxypyrimidin-2-yl)-3-[1-methyl-4-(2-methyl-2H-tetrazol-5-yl)pyrazol-5-ylsulfonyl)harnstoff, 4-(4-Chlor-2-methylphenoxy)buttersäure (Common name: MCPB), 2,4-Bis(ethylamino)-6-methylthio-1,3,5-triazine (Common name: Simetryne), [[(4,6-dimethoxy-2-pyrimidinyl)amino]carbonyl]-2-ethoxyphenylester (Common name: Ethoxysulfuron).

Zu weiteren Wirkstoffen gehören insbesondere Metallsalze des Pflanzenschutzwirkstoffs Quinclorac bzw. der aromatischen Carbonsäure der Formel (V)

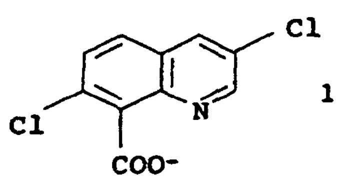 1/n $M^{(n+)}$ (V)

wobei M ein 1-wertiges (n=1) oder 2-wertiges (n=2) Metallkation bedeutet.

Außerdem kann es von Nutzen sein, das Cyclohexenonoximether-Lithiumsalz (I) allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Auch die Verwendung von nach dem erfindungsgemäßen Verfahren herstellbarem 2-{1-[2-(4-Chlorphenoxy)-propoxyimino]-butyl}-5-tetrahydrothiopyran-3-yl-3-hydroxy-cyclohex-2-en-1-on-Lithiumsalz als Herbizid gehört zur vorliegenden Erfindung. Dies betrifft die Verwendung als Pflanzenschutzmittel in der Landwirtschaft gegebenenfalls in Kombination mit anderen Pflanzenschutz- und/oder Düngemitteln.

In Abhängigkeit von der jeweiligen Applikationsmethode kann das Li-Salz (I) in einer Vielzahl von Kultur- und Zierpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spp. altissima, Beta vulagris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Ficus elastica, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana

tabacum (N.rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Bevorzugt ist die Verwendung als Reisherbizid.

Darüber hinaus kann das Cyclohexenonoximether-Lithiumsalz (I) auch in Kulturen verwendet werden, die gegen die Wirkung von Herbiziden tolerant sind.

Die Applikation des erfindungsgemäßen Salzes (I) bzw. deren Aufbereitung kann im Vorauflauf- oder im Nachauflaufverfahren erfolgen, vornehmlich durch Blattspritzung. Ist der Wirkstoff für gewisse Kulturpflanzen weniger verträglich, so können Ausbringungstechniken angewandt werden, bei welchen die Mittel mit Hilfe der Spritzgeräte so gespritzt werden, daß die Blätter der empfindlichen Kulturpflanzen nach Möglichkeit nicht getroffen werden, während der Wirkstoff auf die Blätter darunter wachsender unerwünschter Pflanzen oder die unbedeckte Bodenfläche gelangen (post-directed, lay-by).

Die Aufwandmengen an Wirkstoff (I) betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.). Dabei kann die Ausbringung z.B. mit Wasser als Trägerstoff durch übliche Spritztechniken mit Mengen von etwa 100 bis 1000 l/ha Spritzbrühe erfolgen. Eine Anwendung der herbiziden Mittel im sogenannten "Low Volume"- oder "Ultra-low-Volume"-Verfahren ist ebenso möglich wie ihre Applikation in Form von Granulaten.

Das nach dem erfindungsgemäßen Verfahren herstellbare Cyclohexenonoximether-Li-Salz (I) bildet überraschenderweise vergleichsweise stabilere SC-Formulierungen, insbesondere Öl-SC-Formulierungen. Dies ist vor allem für eine Verwendung als Reisherbizid und einem weiteren Aspekt zufolge für eine Verwendung im tropischen Bereich von Bedeutung. Dabei ist es vorteilhaft, eine vergleichsweise hohe Dauerlagerstabilität bei erhöhter Temperatur im Bereich von 30-40°C gewährleisten zu können.

Die folgenden Beispiele sollen die Erfindung näher erläutern.

Herstellungsbeispiele

Beispiel 1

Einsatzprodukt: Kalziniertes LiOH (Verfahren A)

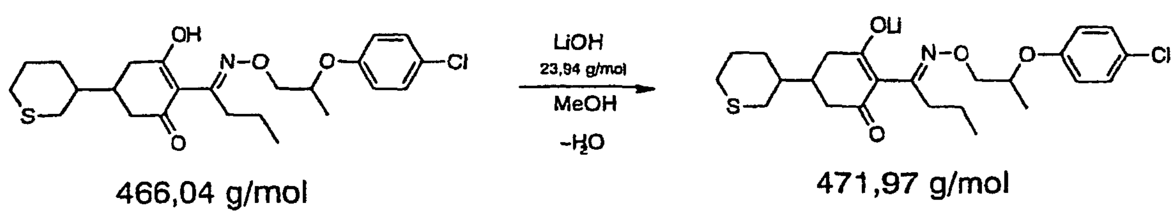


702,78 g (0,400 mol) einer 26,5%igen toluolischen Lösung des Cyclohexenonoximethers (II) werden bei 25°C unter Rühren vorgelegt und mit 229,47 g Toluol verdünnt, wodurch eine Konzentration des Cyclohexenonoxim-ethers (II) von 20% eingestellt wird. Anschließend werden 200,98 g (0,420 mol) einer 5 %igen LiOH-Lösung (kalziniertes LiOH (98+); Chemetall) in MeOH innerhalb von 30 min zugefahren. Es wird 1 h bei 25°C gerührt. Danach werden 370,59 g eines MeOH/Toluol/Wasser-Gemisches bei 50°C im Vakuum abdestilliert. Die Temperatur wird hierbei bei 50°C gehalten und der Druck geregelt von 450 mbar auf 200 mbar abgesenkt. Die Destillationszeit beträgt 210 min. Nach Abschluß der Destillation werden 13 g Methanol zugesetzt, es wird 15 Minuten bei Raumtemperatur gerührt und die Suspension aus dem Rührbehalter abgelassen und auf eine Druckfilternutsche gegeben. Nach beendeter Filtration wird der Rührbehälter mit 174,00 g (200 ml) frischem Toluol ausgespült und der Filterkuchen mit diesem Spültoluol gewaschen. Danach wird der Filterkuchen zweimal mit je 87,0 g (100 ml) frischem Toluol gewaschen. Anschließend wird der Filterkuchen im Vakuumtrockenschrank bei 24 h bei 50°C getrocknet. Erhalten werden 181,01 g Filterkuchen mit einem Gehalt von 98,2%. Unter Berücksichtigung dieses Gehalts beträgt die Ausbeute 94,1%.

Beispiel 2

Einsatzprodukt: LiOH-Monohydrat (Verfahren B)

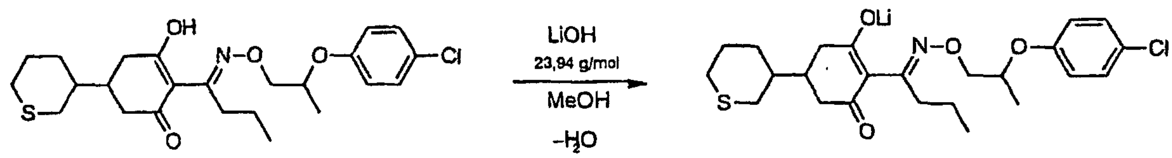


466,04 g/mol    471,97 g/mol

770,73 g (0,400 mol) einer 24,2%igen toluolischen Lösung des Cyclohexenonoximethers (II) werden bei 25°C unter Rühren vorgelegt und mit 161,84 g Toluol verdünnt, wodurch eine Konzentration des Cyclohexenonoximethers (II) von 20% eingestellt wird. Anschließend werden 200,99 g (0,420 mol) einer 8,8%igen LiOH-Monohydrat-Lösung in MeOH (Chemetall; entspricht einer 5%igen Lösung bezogen auf LiOH (100%)) innerhalb von 30 min zugefahren. Es wird 1 h bei 25°C gerührt. Danach werden 381,21 g eines MeOH/Toluol/Wasser-Gemisches bei 50°C im Vakuum abdestilliert. Die Temperatur wird hierbei bei 50°C gehalten und der Druck geregelt von 450 mbar auf 200 mbar abgesenkt. Die Destillationszeit beträgt 230 min. Nach Abschluß der Destillation wird ein Schuß Methanol zugesetzt und die Suspension aus dem Rührbehalter abgelassen und auf eine Druckfilternutsche gegeben. Nach beendeter Filtration wird der Rührbehälter mit 174,00 g (200 ml) frischem Toluol ausgespült und der Filterkuchen mit diesem Spültoluol gewaschen. Danach wird der Filterkuchen zweimal mit je 87,0 g (100 ml) frischem Toluol gewaschen. Anschließend wird der Filterkuchen im Vakuumtrockenschrank bei 24 h bei 50°C getrocknet. Erhalten werden 189,60 g Filterkuchen mit einem Gehalt von 96,5%. Unter Berücksichtigung dieses Gehalts beträgt die Ausbeute 97,0%.

Filtrationseigenschaften und Ausbeuten

Beispiel 3:

Bestimmung der Filterwiderstände und Filtrationszeiten

Die nach der Destillation erhaltene Suspension des Lithiumsalzes in Toluol wird in einen 2,2 Ltr. Druckfilter aus Hastelloy C 4 mit 20 $cm^2$ Filterfläche und einem Filtertuch aus Polypropylen (PP 2703) eingefüllt. Anschließend wird der Druckfilter verschlossen und ein Filtrationsdruck von einem bar aufgegeben. Während des Filtrierens wird der Druck konstant gehalten und die Zeit bis zum Gasdurchbruch gestoppt. Zum Schluß wird entspannt, der Filter geöffnet und die Kuchendicke gemessen.
Tabelle 1 zeigt vergleichend Filterwiderstände, Filtrationszeiten und Ausbeuten für die nach den angegebenen Verfahren hergestellten Cyclohexenonoximether-Lithium-Salze (I).
Der in Tabelle 1 angegebene Filterwiderstand $\alpha*\eta$ berechnet sich nach der Formel:

$$\alpha*\eta = 2*t_F*\text{Filterfläche}*\text{Filtrationsdruck}*\text{Filtratvolumen}^{-1}*\text{Filterkuchenhöhe}^{-1}$$

Tabelle 1:

| Filterwiderstände und Filtrationszeiten | | | | |
|---|---|---|---|---|
| Verfahren | Versuch | Filterwiderstände $\alpha*\eta$ (mPas/$m^2$) | Filtrationszeit $t_F$(s) | Ausbeute (%) |
| *Vergleichsverfahren A* | 1. Versuch | 2,2 x $10^{13}$ | 7274 | 95,9% |
| Cyclohexenonoxim- | 2. Versuch | 5,6 x $10^{13}$ | 18315 | 94,9% |

Tabelle 1: (fortgesetzt)

| Filterwiderstände und Filtrationszeiten | | | | |
|---|---|---|---|---|
| Verfahren | Versuch | Filterwiderstände $\alpha*\eta$ (mPas/ $m^2$) | Filtrationszeit $t_F$(s) | Ausbeute (%) |
| ether (II) + $LiOH/H_2O$ (s. Herstellungsbeispiel 6 der WO 97/20807) | 3. Versuch | 2,0 x $10^{14}$ | 65410 | 94,2% |
| *Vergleichsverfahren B*<br>Cyclohexenonoximether Na-Salz + LiCl (s. Herstellungsbeispiel 9 der WO 97/20807) | 1. Versuch<br>2. Versuch | 3,8 x $10^{11}$<br>4,1 x $10^{11}$ | 138<br>151 | 89,8<br>90,2 |
| *Erfindungsgemäßes Verfahren A*<br>Cyclohexenonoximether (II) + LiOH (98%) in MeOH (s. Herstellungsbeispiel 1) | 1. Versuch<br>2. Versuch<br>3. Versuch | 3,2 x $10^{11}$<br>3,9 x $10^{11}$<br>3,6 x $10^{11}$ | 103<br>115<br>102 | 94,1<br>94,5<br>93,8 |
| *Erfindungsgemäßes Verfahren B*<br>Cyclohexenonoximether (II) + LiOH-Monohydrat (57%) in MeOH (s. Herstellungsbeispiel 2) | 1. Versuch<br>2. Versuch<br>3. Versuch | 3,5 x $10^{11}$<br>2,1 x $10^{11}$<br>3,7 x $10^{11}$ | 107<br>56<br>130 | 97,0<br>96,7<br>95,9 |

Die besten Filtrationseigenschaften und Ausbeuten resultieren, wenn die Cyclohexenonoximether-Li-Salze (I) nach den erfindungsgemäßen Verfahren gemäß Beispiel 1 und 2 hergestellt werden.

Öl-SC-Formulierungen

Beispiel 4

Allgemeine Versuchsbeschreibung zur Herstellung von Öl-SC-Formulierungen

Unter Verwendung von Glasperlen (Durchmesser: 0,9-1,2 mm) als Mahlhilfsmittel werden die Lithium-Salze (I) bei ca. 0°C mit den lipophilen Solventien - hier allgemein flüssige Medien, die keine oder nur eine geringe Lösewirkung gegenüber den Wirkstoffsalzen zeigen, vermahlen. Die Wirkstoffkonzentration beträgt ca. 10-30%, allgemein 20%.

Die Mahlungen erfolgen in einer Dynomühle der Fa. Bachofen mit einer Ansatzgröße von 0,5 bis zu einem Liter in sogenannter Passagenfahrweise. Nach in der Regel 5 Passagen (Durchpumpen des Slurry durch die Mühle mit Hilfe einer Schlauchpumpe) werden dabei nach mikroskopischer Auswertung mittlere Teilchengrößen von 1-3 µm erreicht.

Anschließend erfolgt die Einarbeitung und Verdünnung mit weiteren Hilfsmitteln gemäß unten angegebener Rezepturen durch 10-minütiges Homogenisieren mit KPG- oder Magnetrührern.

Durchführung der Versuche

90 % an Lösemittel (Ester und/oder aromatische Lösemittel) werden mit den angegebenen Hilfsmitteln (Emulgier- und Dispergiermittel) gemäß unten genannter und jeweils identischer Rezeptur vorgelegt und der Wirkstoff (I) als festes Pulver zugegeben.

Anschließend wird der Formulieransatz mit der Restmenge an Lösemittel auf 1 l aufgefüllt (typische Ansatzgröße).

Danach wird der Absatz wie oben beschrieben in einer Dynomühle über Passagenfahrweise gemahlen, bis ca. 60 % der Teilchen eine Größe von weniger 2 µm bei mikroskopischer Auswertung aufweisen. Typischerweise werden dazu 5 Passagen benötigt.

| | | |
|---|---|---|
| Rezeptur | 75 g/l | (I) |
| | 250 g/l | Aerosol OT- A |
| | 50 g/l | Emulpon EL 20 |
| | 500 g/l | Methyloleat |
| | ad 11 | Solvesso 150 |

Tabelle 2 zeigt Daten zur Lagerstabilität der nach den einzelnen Verfahren hergestellten Cyclohexenonoximether-Li-Salze (I).

Tabelle 2:

| Öl-SC-Lagerstabilität in % (rel.) nach 14-28 d bei 54°C | | |
|---|---|---|
| **Verfahren** | **2 Wochen 54°C** | **4 Wochen 54°C** |
| *Vergleichsverfahren A*<br>Cyclohexenonoximether (II) + LiOH/$H_2O$ (s. Herstellungsbeispiel 6 der WO 97/20807) | **93,4** | **88,2** |
| *Vergleichsverfahren B*<br>Cyclohexenonoximether Na-Salz + LiCl (s. Herstellungsbeispiel 9 der WO 97/20807) | **94,2** | **91,2** |
| *Erfindungsgemäßes Verfahren A*<br>Cyclohexenonoximether (11) + LiOH (98%) in MeOH | **95,6** | **94,8** |

Nach einer Lagerzeit von 4 Wochen bei 54°C zeigt sich, dass das beispielsweise nach dem erfindungsgemäßen Verfahren A hergestellte Lithium-Salz eine deutlich bessere Lagerstabilität aufweist als ein vergleichbares nach dem Stand der Technik hergestelltes Lithium-Salz.

**Patentansprüche**

**1.** Verfahren zur Herstellung von 2-{1-[2-(4-Chlorphenoxy)-propoxyimino]-butyl}-3-hydroxy-5-(tetrahydrothiopyran-3-yl)-cyclohex-2-enon-Lithiumsalz der Formel (I),

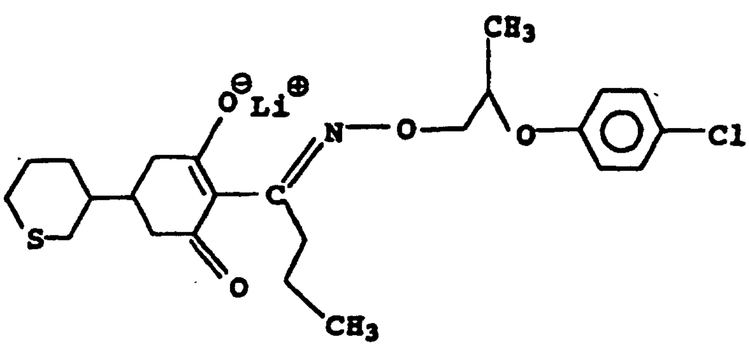

(I)

durch Umsetzung von 2-{1-[2-(4-Chlorphenoxy)-propoxyimino]-butyl}-3-hydroxy-5-(tetrahydrothiopyran-3-yl)-cyclohex-2-enon der Formel (II)

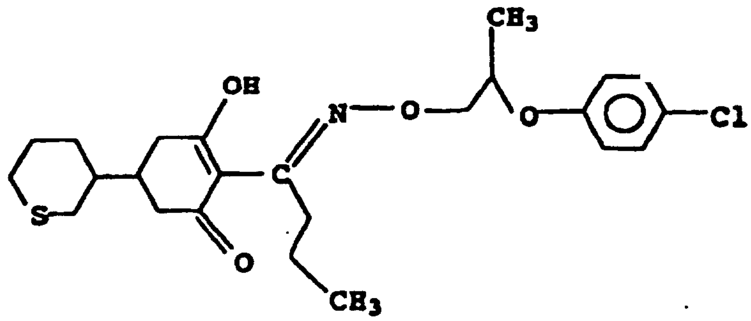

(II)

und Lithiumhydroxid in einem Lösungsmittelgemisch sowie Gewinnung der Verbindung der Formel (I), **dadurch gekennzeichnet, dass** das Lösungsmittelgemisch wenigstens einen aromatischen Kohlenwasserstoff und Methanol enthält und vor Gewinnung wenigstens ein Teil des Lösungsmittels entfernt wird.

2. Verfahren nach Anspruch 1, **dadurch gekennzeichnet, dass** man als aromatischen Kohlenwasserstoff Toluol verwendet.

3. Verfahren nach Anspruch 1 oder 2, **dadurch gekennzeichnet, dass** die Lösung der Verbindung der Formel (II) diese in einer Konzentration von 5 bis 40 Gew.-%, vorzugsweise von 10 bis 30 Gew.-% und insbesondere etwa 20 Gew.-% enthält.

4. Verfahren nach einem der vorhergehenden Ansprüche, **dadurch gekennzeichnet, dass** die Lithiumhydroxid-Lösung LiOH in einer Konzentration von 1 bis 7 Gew.-%, vorzugsweise von 2 bis 6 Gew.-% und insbesondere von etwa 5 Gew.-% enthält.

5. Verfahren nach einem der vorhergehenden Ansprüche, **dadurch gekennzeichnet, dass** die Umsetzung bei Temperaturen von -20°C bis 60°C, vorzugsweise von 0°C bis 40°C und insbesondere von 20 bis 30°C durchgeführt wird.

6. Verfahren nach einem der vorhergehenden Ansprüche, **dadurch gekennzeichnet, dass** das Lösungsmittel durch Destillation entfernt wird.

7. Verfahren nach Anspruch 6, **dadurch gekennzeichnet, dass** die Destillation bei Temperaturen von 20°C bis 70°C, vorzugsweise von 30°C bis 60°C und insbesondere von 40°C bis 50°C durchgeführt wird.

8. Verfahren nach Anspruch 6 oder 7, **dadurch gekennzeichnet, dass** die Destillation bei im wesentlichen konstanter Temperatur durchgeführt wird.

9. Verfahren nach einem der Ansprüche 6 bis 8, **dadurch gekennzeichnet, dass** soviel Lösungsmittel entfernt, dass die Verbindung der Formel (I) im wesentlichen vollständig ausfällt.

10. Verfahren nach einem der vorhergehenden Ansprüche, **dadurch gekennzeichnet, dass** die Gewinnung durch Filtration oder Zentrifugation erfolgt.

## Claims

1. A process for preparing 2-{1-[2-(4-chlorophenoxy)-propoxyimino]butyl}-3-hydroxy-5-(tetrahydrothiopyran-3-yl)-cyclohex-2-enone lithium salt of the formula (I),

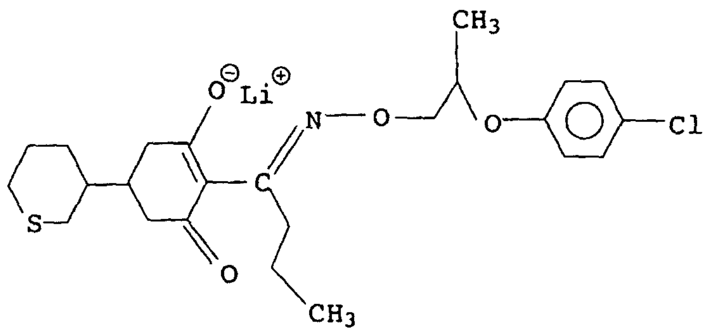

(I)

by reacting 2-{1-[2-(4-chlorophenoxy)propoxyimino]-butyl}-3-hydroxy-5-(tetrahydrothiopyran-3-yl)cyclohex-2-enone of the formula (II)

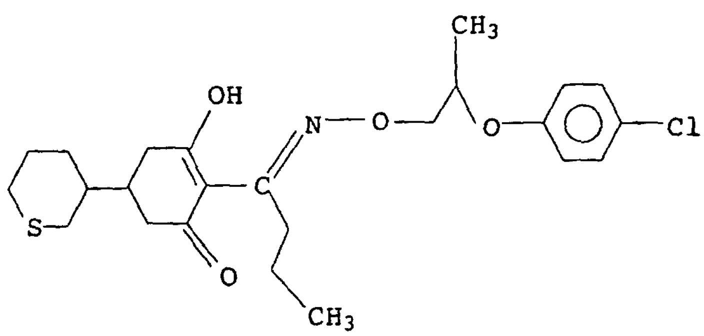

(II)

and lithium hydroxide in a solvent mixture and isolating the compound of the formula (I),
wherein the solvent mixture comprises at least one aromatic hydrocarbon and methanol and at least some of the solvent is removed prior to the isolation.

**2.** A process as claimed in claim 1, wherein the aromatic hydrocarbon used is toluene.

**3.** A process as claimed in claim 1 or 2, wherein the solution of the compound of the formula (II) comprises the compound in a concentration of from 5 to 40% by weight, with preference from 10 to 30% by weight and in particular about 20% by weight.

**4.** A process as claimed in any of the preceding claims, wherein the lithium hydroxide solution comprises LiOH in a concentration of from 1 to 7% by weight, preferably from 2 to 6% by weight and in particular about 5% by weight.

**5.** A process as claimed in any of the preceding claims, wherein the reaction is carried out at temperatures of from -20°C to 60°C, preferably from 0°C to 40°C and in particular from 20 to 30°C.

**6.** A process as claimed in any of the preceding claims, wherein the solvent is removed by distillation.

**7.** A process as claimed in claim 6, wherein the distillation is carried out at temperatures of from 20°C to 70°C, preferably from 30°C to 60°C and in particular from 40°C to 50°C.

**8.** A process as claimed in claim 6 or 7, wherein the distillation is carried out at a substantially constant temperature.

**9.** A process as claimed in any of claims 6 to 8, wherein such an amount of solvent is removed that the compound of the formula (I) precipitates out substantially completely.

**10.** A process as claimed in any of the preceding claims, wherein the isolation is carried out by filtration or centrifugation.

**Revendications**

**1.** Procédé de préparation du sel de lithium de 2-{1-[2-(4-chlorophénoxy)propoxyimino]butyl}-3-hydroxy-5-(tétrahydrothiopyran-3-yl)cyclohex-2-énone de formule (I),

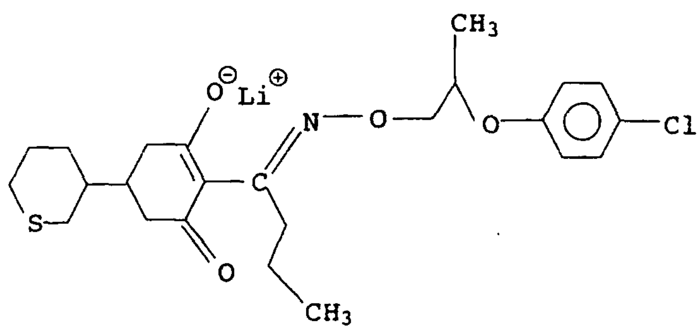

(I)

par réaction de la 2-{1-[2-(4-chlorophénoxy)propoxyimino]butyl}-3-hydroxy-5-(tétrahydrothiopyran-3-yl)cyclohex-2-énone de formule (II)

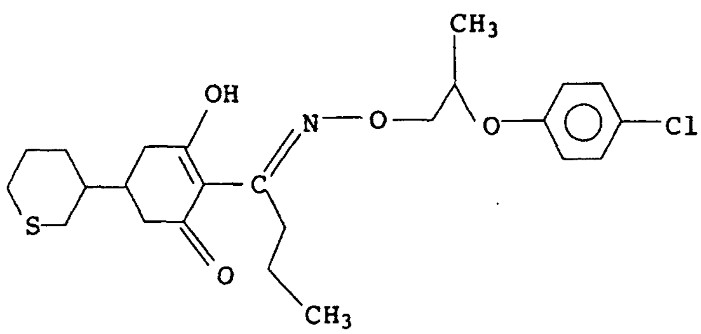

(II)

et de l'hydroxyde de lithium dans un mélange de solvants et isolation du composé de formule (I), **caractérisé en ce que** le mélange de solvants contient au moins un hydrocarbure aromatique et du méthanol et, avant l'isolation, au moins une partie du solvant est éliminée.

**2.** Procédé selon la revendication 1, **caractérisé en ce que** l'on utilise le toluène en tant qu'hydrocarbure aromatique.

**3.** Procédé selon la revendication 1 ou 2, **caractérisé en ce que** la solution du composé de formule (II) contient celui-ci en une concentration de 5 à 40% en poids, de préférence de 10 à 30% en poids et en particulier d'environ 20% en poids.

**4.** Procédé selon l'une quelconque des revendications précédentes, **caractérisé en ce que** la solution d'hydroxyde de lithium contient du LiOH en une concentration de 1 à 7% en poids, de préférence de 2 à 6% en poids et en particulier d'environ 5% en poids.

**5.** Procédé selon l'une quelconque des revendications précédentes, **caractérisé en ce que** la réaction est réalisée à des températures de -20°C à 60°C, de préférence de 0°C à 40°C et en particulier de 20 à 30°C.

**6.** Procédé selon l'une quelconque des revendications précédentes, **caractérisé en ce que** le solvant est éliminé par distillation.

**7.** Procédé selon la revendication 6, **caractérisé en ce que** la distillation est réalisée à des températures de 20°C

à 70°C, de préférence de 30°C à 60°C et en particulier de 40°C à 50°C.

8. Procédé selon la revendication 6 ou 7, **caractérisé en ce que** la distillation est réalisée à une température essentiellement constante.

9. Procédé selon l'une quelconque des revendications 6 à 8, **caractérisé en ce que** le solvant est éliminé jusqu'à ce que le composé de formule (I) précipite essentiellement totalement.

10. Procédé selon l'une quelconque des revendications précédentes, **caractérisé en ce que** l'isolation est réalisée par filtration ou centrifugation.